# EUROPEAN PATENT SPECIFICATION

(11) **EP 4 554 661 B1**
(45) Date of publication and mention of the grant of the patent: **28.10.2026**
(21) Application number: 23782619.3
(22) Date of filing: 27.09.2023
(51) Int. Cl.: A61N 1/36, A61B 5/00, A61B 5/0538, A61B 5/01, A61B 5/25, A61N 1/40, A61B 5/0531, A61N 1/08, A61B 5/07

(54) **INGESTIBLE IMPLANTABLE DEVICE TO MEASURE INTERNAL TTFIELD INTENSITY**
EINNEHMBARE IMPLANTIERBARE VORRICHTUNG ZUR MESSUNG DER INTENSITÄT DES INNEREN TTFIELDS
DISPOSITIF IMPLANTABLE INGÉRABLE POUR MESURER UNE INTENSITÉ DE CHAMP TT INTERNE

(30) Priority: 27.09.2022 US 202263377256 P
(43) Date of publication of application: 21.05.2025
(73) Proprietor: Novocure GmbH, 6340 Baar (CH)
(72) Inventor: WASSERMAN, Yoram, 31905 Haifia (IL)
(74) Representative: Carpmaels & Ransford LLP
(86) International application number: PCT/IB2023/059633
(87) International publication number: WO 2024/069488

(56) References cited:
- CN-U- 217 162 096
- US-A1- 2019 117 972
- US-A1- 2019 117 973
- US-A1- 2021 162 228
- US-A1- 2022 087 593
- US-A1- 2022 225 879

## Description

The present invention relates to an ingestible or implantable probing device adapted to determine a property of tumor treating fields (TTFields) applied to a patient.

### BACKGROUND OF THE INVENTION

Tumor Treating Fields (TTFields or TTFs) are low intensity (e.g., 1-3 V/cm) alternating electric fields within the intermediate frequency range (50 kHz to 1 MHz, such as, for example, 100-500 kHz) that target solid tumors by disrupting mitosis. This non-invasive treatment targets solid tumors and is described, for example, in U.S. Patent Nos. 7,016,725; 7,089,054; 7,333,852; 7,565,205; 8,244,345; 8,715,203; 8,764,675; 10,188,851; and 10,441,776. TTFields are typically delivered through two pairs of transducer arrays that generate perpendicular fields within the treated tumor; the transducer arrays that make up each of these pairs are positioned on opposite sides of the body part that is being treated. TTFields are approved for the treatment of glioblastoma multiforme (GBM), and may be delivered, for example, via the OPTUNE^{®} system (Novocure Limited, St. Helier, Jersey), which includes transducer arrays placed on the patient's shaved head.

Each transducer array used for the delivery of TTFields in the OPTUNE^{®} device comprises a set of non-conductive ceramic disk electrodes, which are coupled to the patient's skin (such as, but not limited to, the patient's shaved head for treatment of GBM) through a layer of conductive medical gel. To form the ceramic disk electrodes, a conductive layer is formed on a top surface of nonconductive ceramic material. A bottom surface of the nonconductive ceramic material is coupled to the conductive medical gel.

One approach to applying the TTField in different directions is to apply the field between a first set of electrodes in a first direction for a period of time, then applying a field between a second set of electrodes in a second direction for a period of time, then repeating that cycle for an extended duration (e.g., over a period of days, weeks, or months). In order to generate the TTFields, current is applied to each electrode of the transducer array.

US-A-2022/0087593 discloses a device for measuring congestion of the digestive tract.

CN-U-217162096 discloses a brain tumor field intensity detection instrument.

US-A-2022/0225879 discloses computer-implemented methods and systems under the control of one or more processors of a medical device, where the one or more processors are configured with specific instructions.

US-A-2019/0117972 and US-A-2019/0117973 disclose medical device systems which include electric field shaping elements for use in treating cancerous tumors within bodily tissue.

### SUMMARY OF THE INVENTION

The TTFields interact with the patient and one or more of the patient's organs based on the electrical impedance, resistance, resistivity, or conductivity of each of the patient's organs. As the TTField interacts with the patient, the field may change shape based in part on the electrical impedance, resistance, resistivity, or conductivity and relative position of each of the patient's organs. Because the electrical conductivity of each organ of a patient modifies the TTField shape and a particular TTField shape may be needed to effectively target a tumor, it is important to be able to determine how the applied TTField is shaped within a patient.

To date, there has not been a way to measure actual TTField shape in a patient without computer simulations; however, computer simulations or other models are reliant on programming techniques and estimations, and cannot show the actual TTField shape expected in a patient.

Because the electrical impedance, resistance, resistivity, or conductivity of each organ of a patient modifies the TTField shape and a particular TTField shape may be needed to effectively target a tumor, new and improved assemblies and devices of determining a magnitude and/or a direction of the TTField are desired. It is to such assemblies and devices of producing and using the same that the present disclosure is directed.

In one aspect the present invention provides an ingestible or implantable device according to claim 1.

Embodiments of the invention are disclosed in the dependent claims.

The details of one or more implementations of the subject matter described in this specification are set forth in the accompanying drawings and the description below. Other aspects, features and advantages of the subject matter will become apparent from the description, the drawings, and the claims.

### BRIEF DESCRIPTION OF THE DRAWINGS

The accompanying drawings, which are incorporated in and constitute a part of this specification, illustrate one or more implementations described herein and, together with the description, explain these implementations. The drawings are not intended to be drawn to scale, and certain features and certain views of the figures may be shown exaggerated, to scale or in schematic in the interest of clarity and conciseness. Not every component may be labeled in every drawing. Like reference numerals in the figures may represent and refer to the same or similar element or function. In the drawings:
FIG. 1 is a schematic diagram of exemplary embodiments of electrodes as applied to a field target such as living tissue and having a probing device implanted within the field target;
FIG. 2 is a schematic diagram of an exemplary embodiment of an electronic device configured to generate a TTField;
FIG. 3 is a schematic diagram of an exemplary embodiment of a transducer array;
FIG. 4 is a schematic diagram of an exemplary embodiment of a probing device;
FIG. 5 is a block diagram of an exemplary embodiment of a controller;
FIG. 6 is a block diagram of an exemplary embodiment of a computer system;
FIG. 7 is a process flow diagram of an exemplary embodiment of a method for determining a property of an electric field; and
FIG. 8 is a process flow diagram of an exemplary embodiment of a method for determining a transducer array arrangement.

### DETAILED DESCRIPTION

It is to be understood that the present invention is not limited to the exemplary embodiments, details and arrangements set forth in the following description, but is defined by the appended claims.

Unless otherwise required by context, singular terms shall include pluralities and plural terms shall include the singular, with the exception that the term "plurality" as used herein, does not include the singular.

All of the assemblies, systems, kits, and/or methods disclosed herein can be made and executed without undue experimentation in light of the present disclosure. This holds for any possible non-express basis for interpretation, including matters of logic with respect to arrangement of steps or operational flow, plain meaning derived from grammatical organization or punctuation, or the number or type of embodiments described in the specification.

As utilized in accordance with the present disclosure, the following terms, unless otherwise indicated, shall be understood to have the following meanings:
The use of the term "a" or "an" when used in conjunction with the term "comprising" in the claims and/or the specification may mean "one," but it is also consistent with the meaning of "one or more," "at least one," and "one or more than one." The term "plurality" refers to "two or more".

The use of the term "at least one" will be understood to include one as well as any quantity more than one. In addition, the use of the term "at least one of X, Y, and Z" will be understood to include X alone, Y alone, and Z alone, as well as any combination of X, Y, and Z.

The use of ordinal number terminology (i.e., "first," "second," "third," "fourth," etc.) is solely for the purpose of differentiating between two or more items and is not meant to imply any sequence or order or importance to one item over another or any order of addition, for example.

The use of the term "or" in the claims is used to mean an inclusive "and/or" unless explicitly indicated to refer to alternatives only or unless the alternatives are mutually exclusive.

The term "patient" as used herein includes human and veterinary subjects. "Mammal" for purposes of treatment refers to any animal classified as a mammal, including (but not limited to) humans, domestic and farm animals, nonhuman primates, and any other animal that has mammary tissue.

Circuitry, as used herein, may be analog and/or digital components, or one or more suitably programmed processors (e.g., microprocessors) and associated hardware and software, or hardwired logic. Also, "components" may perform one or more functions. The term "component," may include hardware, such as a processor (e.g., microprocessor), an application specific integrated circuit (ASIC), a field programmable gate array (FPGA), a combination of hardware and software, and/or the like. The term "processor" as used herein means a single processor or multiple processors working independently or together to collectively perform a task.

As used herein, the term "TTField" (TTFields, or TTF(s)) means tumor treating field. TTFields are low intensity (e.g., 1-4 V/cm) alternating electric fields of medium frequencies (about 50 kHz - 1 MHz, and more preferably from about 50 kHz - 500 kHz) that when applied to a conductive medium, such as a human body, via electrodes, may be used, for example, to treat tumors as described in U.S. Patent Nos. 7,016,725, 7,089,054, 7,333,852, 7,565,205, 7,805,201, and 8,244,345 by Palti and in a publication by Kirson (see Eilon D. Kirson, et al., "Disruption of Cancer Cell Replication by Alternating Electric Fields", Cancer Res. 2004 64:3288-3295). TTFields have been shown to have the capability to specifically affect cancer cells and serve, among other uses, for treating cancer. TTFields therapy is an approved mono-treatment for recurrent glioblastoma (GBM), and an approved combination therapy with chemotherapy for newly diagnosed GBM patients.

As used herein, the term TTSignal(s) is an electrical signal that, when received by electrodes applied to a conductive medium, such as a human body, causes the electrodes to generate the TTField described above. The TT Signal is often an AC electrical signal.

The term "transducer array", as used herein, may mean a conductive transducer array or a non-conductive transducer array. Exemplary transducer arrays may include, for example, transducer arrays disclosed in any one of U.S. Patent Publication No. 2021/0346693 entitled "CONDUCTIVE PAD GENERATING TUMOR TREATING FIELD AND METHODS OF PRODUCTION AND USE THEREOF" and U.S. Patent Publication No. 2022/0193404 entitled "OPTIMIZATION OF COMPOSITE ELECTRODE".

The term "resistance" refers to a degree to which a substance or device, or component of a device, opposes the passage of electric current causing energy dissipation. "Resistivity" is a fundamental property of a substance or material which refers to the degree to which the substance or material opposes the passage of electric current causing energy dissipation, but is standardized: a resistance per unit length and per unit of cross-sectional area at a specified temperature.

The term "impedance" refers to an effective resistance of an electric circuit or component to alternating current, arising from the combined effects of ohmic resistance and reactance.

The term "conductivity" refers to a degree to which a specified material conducts electricity, calculated as the ratio of the current density in the material to the electric field that causes the flow of current. The "conductivity" of a material is the reciprocal of the material's resistivity.

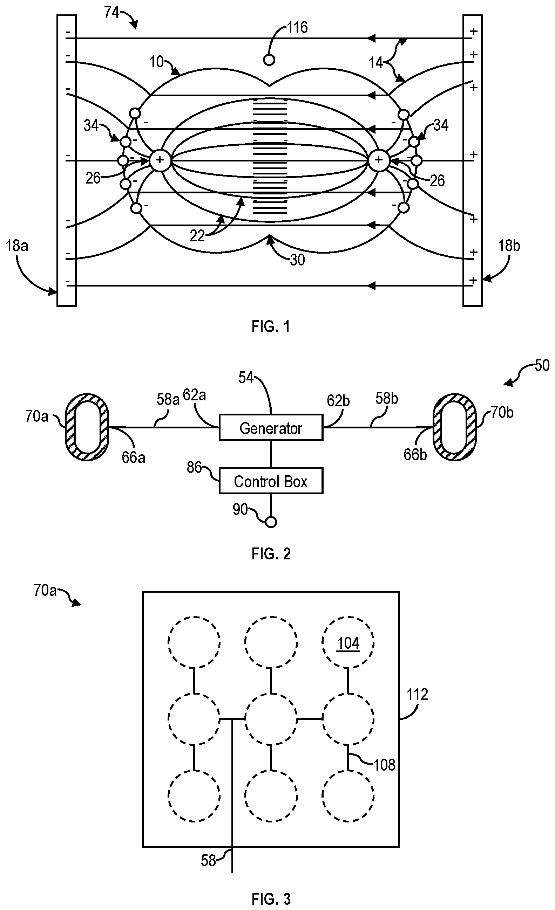

Referring now to the drawings and in particular to FIG. 1, shown therein is a diagram of an exemplary embodiment of a dividing cell 10, under the influence of external TTFields, generally indicated as lines 14, generated by a first electrode 18a having a negative charge and a second electrode 18b having a positive charge. Further shown are microtubules 22 that are known to have a very strong dipole moment. This strong polarization makes the microtubules 22, as well as other polar macromolecules and especially those that have a specific orientation within the cell 10 or its surroundings, susceptible to electric fields. The microtubules' 22 positive charges are located at two centrioles 26 while two sets of negative poles are at a center 30 of the dividing cell 10 and point of attachment 34 of the microtubules 22 to the cell membrane. The locations of the charges form sets of double dipoles and therefore are susceptible to electric fields of differing directions. In one embodiment, the cells go through electroporation, that is, DNA or chromosomes are introduced into the cells using a pulse of electricity to briefly open pores in the cell membranes.

Referring now to FIG. 2, the TTFields described above that have been found to advantageously destroy tumor cells may be generated by an electronic apparatus 50. FIG. 2 is a simple schematic diagram of the electronic apparatus 50 illustrating major components thereof. The electronic apparatus 50 includes an electric field generator 54 and a pair of conductive leads 58, including first conductive lead 58a and second conductive lead 58b. The first conductive lead 58a includes a first end 62a and a second end 62b. The second conductive lead 58b includes a first end 66a and a second end 66b. The first end 62a of the first conductive lead 58a is conductively attached to the electric field generator 54 and the first end 66a of the second conductive lead 58b is conductively attached to the electric field generator 54.

The electric field generator 54 generates desirable electric signals (TTSignals) in the shape of waveforms or trains of pulses as an output. The second end 62b of the first conductive lead 58a is connected to a first transducer array 70a and the second end 66b of the second conductive lead 58b is connected to a second transducer array 70b, that is supplied with the electric signals (e.g., wave forms).

Each of the first transducer array 70a and the second transducer array 70b are in contact with, or otherwise associated with, a field target 74 (see FIG. 1) such as living tissue (e.g., a patient) or a phantom made of material(s) having similar conductive properties as living tissue). The electric signals generate an electric field (i.e., TTField) that is capacitively coupled into the field target 74, the TTField having a frequency and an amplitude, to be generated between the first transducer array 70a and the second transducer array 70b in the field target 74. In one embodiment, the field target 74 is a phantom generally comprising two or more conductive elements.

Each of the first transducer array 70a and the second transducer array 70b include one or more conductive electrode element that may be capacitively coupled with the field target 74 by a non-conductive layer. Alternative constructions for the first transducer array 70a and the second transducer array 70b may also be used, including, for example, transducer arrays using a non-conductive layer formed of a ceramic element that is disc shaped, or is not disc-shaped, and/or non-conductive layer(s) that use non-ceramic dielectric materials positioned over a plurality of flat conductors. Examples of the latter include polymer films disposed over electrical contacts on a printed circuit board or over flat pieces of metal.

In some embodiments, the first transducer array 70a and the second transducer array 70b may also include electrode elements that are not capacitively coupled with the field target 74. In this situation, each of the first transducer array 70a and the second transducer array 70b may be implemented using a region of a conductive material that is configured for placement against a person's body, with no insulating dielectric layer disposed between the conductive elements and the body. Examples of the conductive material include, but are not limited to, a conductive film, a conductive fabric, and/or a conductive foam. Other alternative constructions for implementing the first transducer array 70a and the second transducer array 70b may also be used, as long as they are capable of delivering TTFields to the field target 74. Optionally, a skin-contact layer may be disposed between the first transducer array 70a and the field target 74; and the second transducer array 70b and the field target 74 in any of the embodiments described herein. The skin-contact layer helps to adhere/affix the first transducer array 70a and the second transducer array 70b to the field target 74, provides a conductive pathway for the electric fields to pass between the first and second transducer arrays 70a and 70b and the field target 74 through an intervening non-conductive or conductive layer, and is biocompatible. Examples of skin-contact layers include hydrogel as well as carbon conductive adhesive composites. The latter adhesives may comprise conductive particles, such as, for example, carbon black powder or carbon fibers, etc.

While the electronic apparatus 50 shown in FIG. 2 comprises only two transducer arrays 70 (the first transducer array 70a and the second transducer array 70b), yet, in some embodiments, the electronic apparatus 50 may comprise more than two transducer arrays 70.

The electric field generator 54 generates an alternating voltage wave form at frequencies in the range from about 50 kHz to about 1MHz (preferably from about 100 kHz to about 500 kHz, or from about 100 kHz to about 300 kHz) (i.e., the TTFields). The required voltages are such that an electric field intensity in tissue within the treatment area is in the range of about 0.1 V/cm to about 10 V/cm, such as, for example, 1-4 V/cm. To achieve this field, the potential difference between two conductors 18 (not shown) of the first transducer array 70a and the second transducer array 70b is determined by the relative impedances of the system components, i.e., a fraction of the electric field on each component is given by that component's impedance divided by a total circuit impedance.

In order to optimize the electric field (i.e., TTField) distribution, the first transducer array 70a and the second transducer array 70b (pair of transducer arrays 70) may be configured or oriented differently depending upon the application in which the pair of transducer array 70a and 70b are to be used. The pair of transducer arrays 70a and 70b, as described herein, are externally applied to the field target 74. When the field target 74 is a patient, the pair of transducer arrays 70 may be applied to the patient's skin, in order to apply the electric current, and electric field (TTField), thereby generating current within the patient's tissue. Generally, the pair of transducer arrays 70 are placed on the patient's skin by a user (or helper) such that the electric field is generated across patient tissue within a treatment area. TTFields that are applied externally can be of a local type or widely distributed type, for example, the treatment of skin tumors and treatment of lesions close to the skin surface, or a tumor further in the body.

In one embodiment, the user may be a medical professional, such as a doctor, nurse, therapist, or other person acting under the instruction of a doctor, nurse, or therapist. In another embodiment, the user may be the patient, that is, the patient (and/or a helper) may place the transducer array 70a and the transducer array 70b on their treatment area.

Optionally and according to another exemplary embodiment, the electronic apparatus 50 includes a control box 86 and a temperature sensor 90 coupled to the control box 86, which are included to control the amplitude of the electric field so as not to generate excessive heating in the treatment area.

When the control box 86 is included, the control box 86 controls the output of the electric field generator 54, for example, causing the output to remain constant at a value preset by the user. Alternatively, the control box 86 sets the output at the maximal value that does not cause excessive heating of the treatment area. In either of the above cases, the control box 86 may issue a warning, or the like, when a temperature of the treatment area (as sensed by temperature sensor 90) exceeds a preset limit. The temperature sensor 90 may be mechanically connected to and/or otherwise associated with the first transducer array 70a or the second transducer array 70b so as to sense the temperature of the field target 74 at either one or both of the first transducer array 70a or the second transducer array 70b.

In one embodiment, the control box 86 may turn off, or decrease power of the TTSignal generated by the electrical field generator 54, if a temperature sensed by the temperature sensor 90 meets or exceeds a comfortability threshold. In one embodiment, the comfortability threshold is the temperature at which a patient would be made uncomfortable while using the transducer array 70a and the transducer array 70b. In one embodiment, the comfortability threshold is a temperature at or about 40 degrees Celsius. In one embodiment, the comfortability threshold is a temperature of between about 39 degrees Celsius and 42 degrees Celsius, or a specific selected temperature between about 39 degrees Celsius and 42 degrees Celsius, such as, for example, 41 degrees Celsius.

The conductive leads 58 are standard isolated conductors with a flexible metal shield, preferably grounded thereby preventing spread of any electric field generated by the conductive leads 58. The transducer array 70a and the transducer array 70b may have specific shapes and positioning so as to generate the TTField of a desired configuration, direction, and intensity at the treatment area and only at the treatment area so as to focus the treatment.

The specifications of the electronic apparatus 50 as a whole and its individual components are largely influenced by the fact that at the frequency of the TTFields living systems behave according to their "Ohmic", rather than their dielectric properties.

Referring now to FIG. 3, shown therein is a diagram of an exemplary embodiment of the transducer array 70a constructed in accordance with the present disclosure. The transducer array 70b may be similar in construction and function as the transducer array 70a. for this reason, only the transducer array 70a will be described herein for purposes of brevity. The transducer array 70a includes one or more electrode element 104. As shown in FIG. 3, each transducer array 70a is configured as a set of one or more electrode elements 104. The transducer array 70a may utilize electrode elements 104 that are capacitively coupled. In the example shown in FIG. 3, the transducer array 70a is configured as multiple electrode elements 104 (for example, about 2 cm in diameter) that are interconnected via flex wires 108. Each electrode element 104 may include a ceramic disk and an electrode layer. In one embodiment, the transducer array 70a includes an outer peripheral edge 112.

Alternative constructions for the transducer array 70a may be used, including, for example ceramic elements that are disc-shaped, ceramic elements that are not disc-shaped, and non-ceramic dielectric materials positioned between the electrode layer and a skin-facing surface of the transducer array 70a over a plurality of flat conductors. Examples of non-ceramic dielectric materials positioned over a plurality of flat conductors include: polymer films disposed over electrodes on a printed circuit board or over flat pieces of metal.

In one embodiment, the transducer array 70a may utilize electrode elements 104 that are not capacitively coupled. In this situation, each electrode element 104 of the transducer array 70a would be implemented using a region of a conductive material that is configured for placement against a person's body, with no insulating dielectric layer disposed between the electrode elements 104 and the body. Examples of the conductive material include a conductive film, a conductive fabric, and a conductive foam. Other alternative constructions for implementing the transducer array 70a may also be used, as long as they are capable of delivering TTFields to the person's body. Optionally, a gel layer may be disposed between the transducer array 70a and the person's body in any of the embodiments described herein.

In one embodiment, the transducer array 70a may be constructed in accordance with any transducer array or pad disclosed in U.S. Patent Publication No. 2023/0024216 entitled "CONDUCTIVE PAD GENERATING TUMOR TREATING FIELD AND METHODS OF PRODUCTION AND USE THEREOF". In some embodiments, the electric field generator 54 uses a control process for controlling the voltage and/or current supplied to the transducer array 70a without obtaining or requiring feedback regarding the temperature of the transducer array 70a. In these embodiments, the transducer array 70a may be devoid of a temperature sensor. Additionally, in these embodiments, the leads 58a and/or 58b contain wiring related to powering the electrode elements 104 but are devoid of wires used for temperature measurement. This has the added benefit of reducing the total number of wires extending from the electric field generator 54 to the patient-which improves patient comfort and reduces the level of circuitry as compared to prior art methods which require temperature measurement(s) to prevent the transducer arrays or pads from overheating.

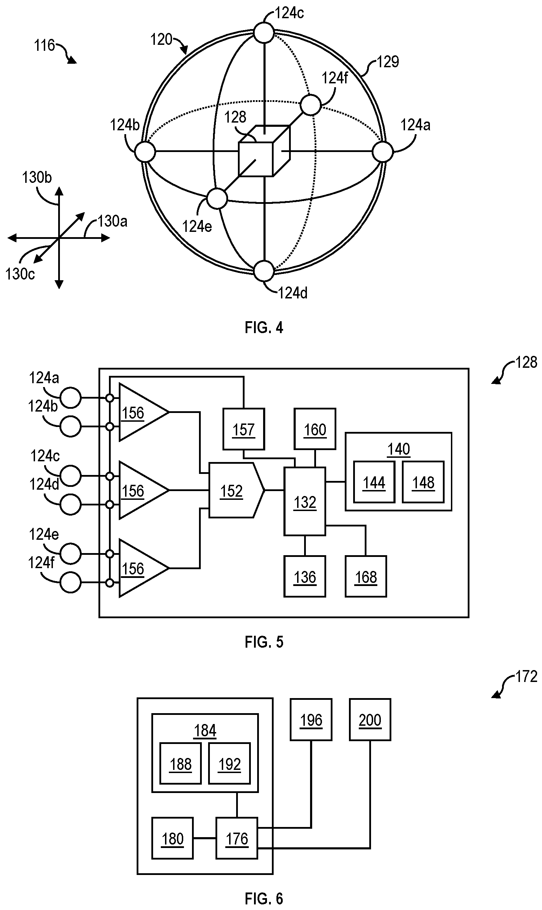

Referring now to FIG. 4, shown therein a schematic diagram of an exemplary embodiment of a probing device 116 constructed in accordance with the present disclosure. As shown in FIG. 1, the probing device 116 can be implanted within the field target 74. In general, the probing device 116 generally comprises a housing 120, a plurality of electrodes 124a-n (collectively "the electrodes 124") supported by the housing 120, and a controller 128 supported within the housing 120. The housing 120 may have a biocompatible outer surface 129; that is, the housing 120 may be configured to be ingestible by a patient and/or implantable into a patient (i.e., surgically implanted into a patient's body). While FIG. 4 depicts the probing device 116 as being spherical in shape, it will be understood by persons having ordinary skill in the art that the probing device 116 may be any shape that is capable of being ingested by a patient and/or implanted into the field target 74 or patient. While FIG. 4 depicts the probing device 116 as comprising six electrodes 124a-f, it will be understood by persons having ordinary skill in the art that the probing device 116 may comprise more than six electrodes 124, such as 8, 10, or 12 electrodes. As will be discussed in greater detail below, each of the electrodes 124 may be connected to the controller 128 for communicating data between the electrodes 124 and the controller 128.

In certain embodiments, the electrodes 124 are grouped into pairs of electrodes 124; that is, the electrodes 124 may comprise a first pair of electrodes 124a-b, a second pair of electrodes 124c-d, and a third pair of electrodes 124e-f. In each pair of electrodes 124, such as the first pair of electrodes 124a-b, a first electrode 124a and a second electrode 124b may be supported by the outer surface 129 of the housing 120 at antipodal points of the housing 120 along a first axis 130a. Similarly, a third electrode 124c and a fourth electrode 124d may be supported by the outer surface 129 of the housing 120 at antipodal points of the housing 120 along a second axis 130b, and a fifth electrode 124e and a sixth electrode 124f may be supported by the outer surface 129 of the housing 120 at antipodal points of the housing 120 along a third axis 130c. The first axis 130a, the second axis 130b, and the third axis 130c may be pairwise perpendicular.

Referring now to FIG. 5, shown therein is a block diagram of an exemplary embodiment of the controller 128 constructed in accordance with the present disclosure. The controller 128 generally comprises one or more processor 132 (hereinafter "the controller processor 132"), one or more communication device 136 (hereinafter "the controller communication device 136"), and one or more non-transitory computer-readable medium 140 (hereinafter "the controller memory 140"). The controller communication device 136 may be configured to communicate using a wireless communication protocol such as, for example, a wireless communication protocol conforming to the requirements of WiFi, Bluetooth, and/or any other wireless communication standard created and/or maintained by the Institute of Electrical and Electronic Engineers (IEEE). As will be described in greater detail below, the controller processor 132 may use the controller communication device 136 to communicate data with a computer system 172, for example. The controller memory 140 may store, for example, processor-executable code 144 (hereinafter "the controller program logic 144") and/or one or more data store 148 (hereinafter "the controller data store 148") for storing, for example, the data received from the electrodes 124. As will be described in greater detail below, the controller program logic 144, when executed, may cause the controller processor 132 to perform one or more step of the methodology described herein for determining a property of an electric field.

The controller 128 may comprise an analog-to-digital converter 152 electrically coupled to one or more amplifier 156 (hereinafter "the amplifiers 156"). In certain embodiments, each pair of electrodes 124 is electrically coupled to one of the amplifiers 156. Each of the amplifiers 156 may be configured to amplify an electric signal received from the electrodes 124, while the analog-to-digital converter 152 may be configured to convert the amplified electrical signal received from each of the amplifiers 156 into a digital signal for delivery to the controller processor 132.

The controller 128 may comprise one or more power source 160 (hereinafter "the power source 160") such as, for example, a battery. The power source 160 may be configured to supply power to one or more component of the controller 128 described herein. The controller 128 further comprises one or more orientation sensors 168 (hereinafter "the orientation sensors 168") configured to measure an orientation of the probing device 116. The orientation sensors 168 may be implemented as an accelerometer, a gyroscope, a magnetometer, and/or combinations thereof, for example. In other embodiments, the orientation sensors 168 are separate from the controller 128 and are supported in a known location within the housing 120.

Generally, the controller program logic 144 when executed causes the controller processor 132 to: measure a first potential difference between the first electrode 124a and the second electrode 124b, the first electrode 124a and the second electrode 124b being spaced a first predetermined distance apart; and transmit, with the controller communication device 136, at least one of first data indicative of the first potential difference and second data indicative of a first property of an electric field based at least in part on the first potential difference (i.e., the first data) and the first predetermined distance. In certain embodiments, the second data is indicative of a magnitude of the electric field and is based at least in part on a quotient determined by dividing the first potential difference (i.e., the first data) by the first predetermined distance. In certain embodiments, the controller program logic 144 when executed further causes the controller processor 132 to store at least one of the first data and the second data in the controller data store 148.

In certain embodiments, the controller program logic 144 when executed further causes the controller processor 132 to: measure a second potential difference between the third electrode 124c and the fourth electrode 124d, the third electrode 124c and the fourth electrode 124d being spaced a second predetermined distance apart; measure a third potential difference between the fifth electrode 124e and the sixth electrode 124f, the fifth electrode 124e and the sixth electrode 124f being spaced a third predetermined distance apart; and transmit, with the controller communication device 136, at least one of third data indicative of the second potential difference, fourth data indicative of the third potential difference, and fifth data indicative of a second property of an electric field based at least in part on the first data, the third data, the fourth data, the first predetermined distance, the second predetermined distance, and the third predetermined distance. In certain embodiments, the controller program logic 144 when executed further causes the controller processor 132 to store at least one of the third data, the fourth data, and the fifth data in the controller data store 148.

In some embodiments, the controller 128 also includes a switching device 157 connected to the controller processor 132, the electrodes 124a-124f, and the inputs of the amplifiers 156. The switching device 157 permits any of the electrodes 124a-f to be connected to the inputs of any of the amplifiers 156 under the control of the controller program logic 144 being executed by the controller processor 132 to permit detection of potential differences in different orientations. For example, it will be understood by persons having ordinary skill in the art that the first, second, and third potential differences and the first, second, and third predetermined distances each may be measured between any two of the electrodes 124 by sending control signals to the switching device 157 to connect any two of the electrodes 124 to the inputs of a particular amplifier 156. For example, the first potential difference may be measured between the first electrode 124a and the fourth electrode 124d, wherein the first predetermined distance is the distance between the first electrode 124a and the fourth electrode 124d; the second potential difference may be measured between the second electrode 124b and the fifth electrode 124e, wherein the second predetermined distance is the distance between the second electrode 124b and the fifth electrode 124e; and the third potential difference may be measured between the third electrode 124c and the sixth electrode 124f, wherein the third predetermined distance is the distance between the third electrode 124c and the sixth electrode 124f. The predetermined distances between any two of the electrodes 124 can be stored in the controller memory 140.

In certain embodiments, the controller program logic 144 when executed further causes the controller processor 132 to: measure, with at least one of the orientation sensors 168, an orientation of the orientation sensor 168; and transmit, with the controller communication device 136, sixth data indicative of the orientation. In certain embodiments, the controller program logic 144 when executed further causes the controller processor 132 to store the sixth data in the controller data store 148. The pairs of electrodes 124 can be fixed in known locations / orientations to the orientation sensor 168 so that the orientation of the electric field between the pairs of electrodes 124 can be determined.

Referring now to FIG. 6, shown therein is a block diagram of an exemplary embodiment of the computer system 172 constructed in accordance with the present disclosure. The computer system 172 generally comprises one or more processor 176 (hereinafter "the computer processor 176"), one or more communication device 180 (hereinafter "the computer communication device 180"), and one or more non-transitory computer-readable medium 184 (hereinafter "the computer memory 184"). The computer communication device 180 may be configured to communicate using a wireless communication protocol such as, for example, a wireless communication protocol conforming to the requirements of WiFi, Bluetooth, and/or any other wireless communication standard created and/or maintained by the Institute of Electrical and Electronic Engineers (IEEE). As will be described in greater detail below, the computer processor 176 may use the computer communication device 180 to communicate data with the controller 128, for example. The computer memory 184 may store, for example, processor-executable code 188 (hereinafter "the computer program logic 188") and one or more data store 192 (hereinafter "the computer data store 192") for storing, for example, the data received from the controller 128. As will be described in greater detail below, the computer program logic 188, when executed, may cause the computer processor 176 to perform one or more step of the methodology described herein for determining a property of an electric field.

The computer system 172 may comprise one or more input device 196 (hereinafter "the input device 196") and one or more output device 200 (hereinafter "the output device 200"). The input device 196 may be implemented as a keyboard, a touchscreen, a mouse, a trackball, a microphone, a fingerprint reader, an infrared port, a cell phone, a personal digital assistant (PDA), a controller, a network interface, speech recognition system, gesture recognition system, eye-tracking system, brain-computer interface system, and/or combinations thereof, for example. The output device 200 may be implemented as of a computer monitor, a screen, a touchscreen, a speaker, a website, a television set, an augmented reality system, a smart phone, a personal digital assistant (PDA), a cell phone, a fax machine, a printer, a laptop computer, an optical head-mounted display (OHMD), a hologram, and/or combinations thereof, for example. The computer system 172 may be implemented as a desktop computer, a laptop computer, a smartphone, a computer tablet, a computer kiosk, or other computing device, for example.

The computer program logic 188 when executed may cause the computer processor 176 to, responsive to receiving data (i.e., the first data indicative of the first potential difference, the second data indicative of the first property of the electric field, the third data indicative of the second potential difference, the fourth data indicative of the third potential difference, the fifth data indicative of the second property of an electric field, and/or the sixth data indicative of the orientation) from the controller 128 with the computer communication device 180, store the data in the computer data store 192, analyze the data, and/or render information onto the one or more output device 200 for viewing by an operator.

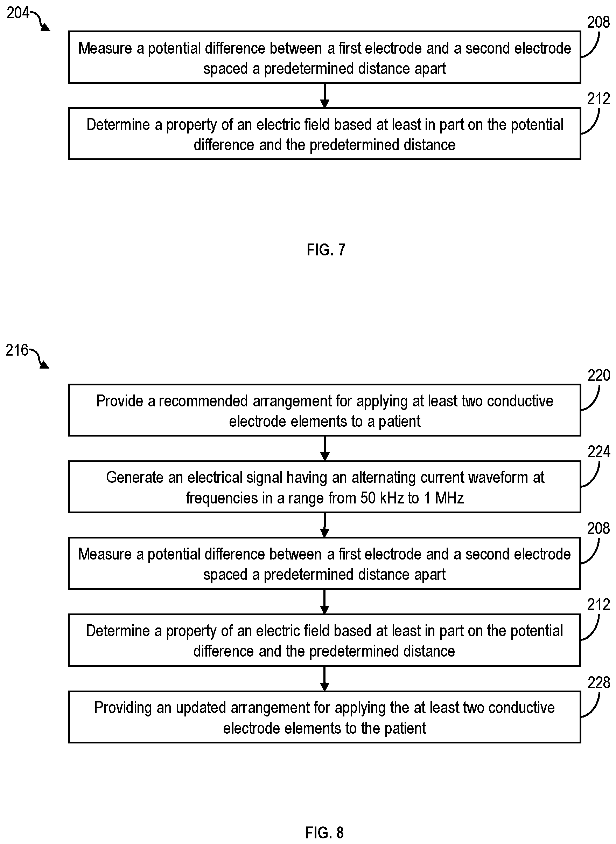

Referring now to FIG. 7, shown therein is a process flow diagram of an exemplary method 204 for determining a property of an electric field as disclosed herein. The method 204 generally comprises the steps of: measuring a potential difference *Vₓ* between the first electrode 124a and the second electrode 124b spaced a predetermined distance *dₓ* apart (step 208); and determining a property of an electric field based at least in part on the potential difference *Vₓ* and the predetermined distance *dₓ* (step 212). The potential difference *Vₓ* may be measured in, for example, volts. The predetermined distance *dₓ* may be measured in, for example, centimeters. In certain embodiments, the method 204 further comprises, prior to measuring the potential difference (step 208), placing the probe device 116 into the field target 74 or patient. The method 204 further comprises measuring the orientation of at least one of the orientation sensors 168.

In certain embodiments, determining the property of the electric field (step 212) is further defined as determining a magnitude *E* and/or direction of the electric field based at least in part on a quotient determined by dividing the potential difference *Vₓ* by the predetermined distance *dₓ* (i.e., $E = \frac{{V}_{x}}{{d}_{x}}$). The magnitude *E* may be measured in, for example, volts per centimeter.

In certain embodiments, the potential difference *Vₓ* is a first potential difference *V*₁, the predetermined distance *dₓ* is a first predetermined distance *d*₁, and the method 204 further comprises the steps of: measuring a second potential difference *V*₂ between the third electrode 124c and the fourth electrode 124d spaced a second predetermined distance *d*₂ apart; and measuring a third potential difference *V*₃ between the fifth electrode 124e and the sixth electrode 124f spaced a third predetermined distance *d*₃ apart. In such embodiments, determining the property of the electric field (step 212) may be further defined as determining the property of the electric field based at least in part on the first potential difference *V*₁, the second potential difference *V*₂, the third potential difference *V*₃, the first predetermined distance *d*₁, the second predetermined distance *d*₂, and the third predetermined distance *d*₃.

In certain embodiments, determining the property of the electric field (step 212) is further defined as determining a direction *θ* of the electric field based at least in part on a vector *̅E̅*̅ = (*Eₓ, E_{y}, E_{z}*) having a first component *Eₓ* based at least in part on the first potential difference *V*₁ and the first predetermined distance *d*₁, a second component *E_{y}* based at least in part on the second potential difference *V*₂ and the second predetermined distance *d*₂, and a third component *E_{z}* based at least in part on the third potential difference *V*₃ and the third predetermined distance *d*₃. The first component *Eₓ* may be based at least in part on a quotient determined by dividing the first potential difference *V*₁ by the first predetermined distance *d*₁ (i.e., ${E}_{x} = \frac{{V}_{1}}{{d}_{1}}$), the second component *E_{y}* may be based at least in part on a quotient determined by dividing the second potential difference *V*₂ by the second predetermined distance *d*₂ (i.e., ${E}_{y} = \frac{{V}_{2}}{{d}_{2}}$), and the third component *E_{z}* may be based at least in part on a quotient determined by dividing the third potential difference *V*₃ by the third predetermined distance *d*₃ (i.e., ${E}_{z} = \frac{{V}_{3}}{{d}_{3}}$). Determining the property of the electric field (step 212) may be performed by one or more of the systems and/or devices described herein.

Referring now to FIG. 8, shown therein is an exemplary method 216 for determining an arrangement for the transducer arrays 70a and 70b. The method 216 generally comprises the steps of, prior to measuring the potential difference (step 208): providing a recommended arrangement for applying at least two conductive electrode elements (i.e., transducer arrays 70a and 70b) to a patient (step 220); and generating an electrical signal having an alternating current waveform at frequencies in a range from 50 kHz to 1 MHz (step 224); and, subsequent to determining the property of the electric field (step 212) with the probing device 216, providing an updated arrangement for applying the at least two conductive electrode elements (i.e., transducer arrays 70a and 70b) to the patient, the updated arrangement based at least in part on the property of the electric field (step 228). The recommended arrangement may be based at least in part on, for example, estimates of TTField intensity in various regions of the patient's body.

Returning now to FIG. 5, the controller program logic 144 may cause the controller processor 132 to transition between a standby mode and a measurement mode in response to receiving instructions from the computer system 172 with the controller communication device 136. While the controller processor 132 is in the standby mode, the controller program logic 144 may cause the controller processor 132 to await instructions, and in some embodiments, idle in a low-power state. Responsive to receiving, with the controller communication device 136, a first instruction to transition into the measurement mode, the controller program logic 144 may cause the controller processor 132 to transition into the measurement mode. While the controller processor 132 is in the measurement mode, the controller program logic 144 may cause the controller processor 132 to perform one or more step of the methodology described herein for determining a property of an electric field. In certain embodiments, the controller program logic 144 may then cause the controller processor 132 to transition into the standby mode. However, as will be described in greater detail below, in other embodiments, the controller program logic 144 may cause the controller processor 132 to transition into the standby mode in response to receiving, with the controller communication device 136, a second instruction to transition into the standby mode.

Returning now to FIG. 6, while the controller processor 132 is in the standby mode, the computer program logic 188 may cause the computer processor 176 to transmit, with the computer communication device 180, the first instruction to transition into the measurement mode. The computer program logic 188 may cause the computer processor 176 to transmit the first instruction in response to a user input, for example. While the controller processor 132 is in the measurement mode, the computer program logic 188 may cause the computer processor 176 to wait to receive data (i.e., the first data indicative of the first potential difference, the second data indicative of the first property of the electric field, the third data indicative of the second potential difference, the fourth data indicative of the third potential difference, the fifth data indicative of the second property of an electric field, and/or the sixth data indicative of the orientation) with the computer communication device 180. In certain embodiments, responsive to receiving such data, the computer program logic 188 may cause the computer processor 176 to transmit the second instruction to transition into the standby mode.

From the above description, it is clear that the inventive concepts disclosed and claimed herein are well adapted to carry out the objects and to attain the advantages mentioned herein, as well as those inherent in the invention. While exemplary embodiments have been described for purposes of this disclosure, it will be understood that numerous changes may be made which will readily suggest themselves to those skilled in the art and which are accomplished within the scope of the invention which is defined by the appended claims.

## Claims

1. An ingestible or implantable probing device adapted to determine a property of tumor treating fields, TTFields, applied to a patient, comprising:
a housing (120) having a biocompatible outer surface (129);
one or more orientation sensors (168) supported in a known location within the housing (120);
a plurality of electrodes (124) supported by the housing (120); and
a controller (128) supported within the housing (120), the controller (128) comprising a processor (132), a communication device (136), and a non-transitory computer-readable medium (140) storing processor-executable code (144) that, when executed, is configured to cause the processor (132) to:
measure, with at least one of the one or more orientation sensors (168), an orientation of the at least one of the one or more orientation sensors (168); measure a potential difference (Vₓ) between a first electrode (124a) and a second electrode (124b) of the plurality of electrodes (124), the first electrode (124a) and the second electrode (124b) being spaced a predetermined distance (dₓ) apart; and
transmit, with the communication device (136), data indicative of the potential difference (Vₓ) and data indicative of the orientation.

2. The device of claim 1, wherein the processor-executable code (144), when executed, is further configured to cause the processor (132) to transmit, with the communication device (136), at least one of the data indicative of the potential difference (Vₓ) and data indicative of a property of an electric field based at least in part on the data indicative of the potential difference (Vₓ) and the predetermined distance (dₓ).

3. The device of claim 2, wherein the first electrode (124a) and the second electrode (124b) are supported by the biocompatible outer surface (129) at antipodal points of the housing (120), and the transmitting comprises transmitting at least one of the data indicative of the potential difference (Vₓ) and data indicative of a magnitude of the electric field based at least in part on a quotient determined by dividing the data indicative of the potential difference (Vₓ) by the predetermined distance (dₓ).

4. The device of claim 1, wherein the potential difference (Vₓ) is a first potential difference (V₁), the predetermined distance (dₓ) is a first predetermined distance (d₁), and the processor-executable code (144), when executed, is further configured to cause the processor (132) to:
measure a second potential difference (V₂) between a third electrode (124c) and a fourth electrode (124d) of the plurality of electrodes (124), the third electrode (124c) and the fourth electrode (124d) being spaced a second predetermined distance (d₂) apart;
measure a third potential difference (V₃) between a fifth electrode (124e) and a sixth electrode (124f) of the plurality of electrodes (124), the fifth electrode (124e) and the sixth electrode (124f) being spaced a third predetermined distance (d₃) apart; and
transmit, with the communication device (136), at least one of data indicative of the second potential difference (V₂) and data indicative of the third potential difference (V₃).

5. The device of claim 4, wherein the processor-executable code (144), when executed, is further configured to cause the processor (132) to transmit, with the communication device (136), data indicative of a property of an electric field based at least in part on the data indicative of the first potential difference (V₁), the data indicative of the second potential difference (V₂), the data indicative of the third potential difference (V₃), the first predetermined distance (d₁), the second predetermined distance (d₂) and the third predetermined distance (d₃).

6. The device of claim 4, wherein the first electrode (124a) and the second electrode (124b) are supported by the biocompatible outer surface (129) along a first axis (130a) of the housing (120), the third electrode (124c) and the fourth electrode (124d) are supported by the biocompatible outer surface (129) along a second axis (130b) of the housing (120), the fifth electrode (124e) and the sixth electrode (124f) are supported by the biocompatible outer surface (129) along a third axis (130c) of the housing (120), and the first axis (130a), the second axis (130b) and the third axis (130c) are pairwise perpendicular.

7. The device of claim 1, where spherical in shape.

8. The device of claim 1, comprising at least six electrodes (124), optionally grouped in pairs.

9. A system, comprising:
the probing device (116) of any of claims 1 to 8; and
a computer system (172) comprising a processor (176), a communication device (180), and a non-transitory computer-readable medium (184) storing processor-executable code (188) that, when executed, is configured to cause the processor (176) of the computer system (172) to:
responsive to receiving the data indicative of the potential difference (Vₓ) with the communication device (180) thereof, store data indicative of a property of an electric field based at least in part on the data indicative of the potential difference (Vₓ) and the predetermined distance (dₓ).

10. The system of claim 9, wherein the first electrode (124a) and the second electrode (124b) are supported by the biocompatible outer surface (129) at antipodal points of the housing (120), and the storing comprises storing data indicative of a magnitude of the electric field based at least in part on a quotient determined by dividing the potential difference (Vₓ) by the predetermined distance (dₓ).

11. The system of claim 9, wherein the potential difference (Vₓ) is a first potential difference (V₁), the predetermined distance (dₓ) is a first predetermined distance (d₁), and the processor-executable code (144) of the probing device (116), when executed, is further configured to cause the processor (132) of the probing device (116) to:
measure a second potential difference (V₂) between a third electrode (124c) and a fourth electrode (124d) of the plurality of electrodes (124), the third electrode (124c) and the fourth electrode (124d) being spaced a second predetermined distance (d₂) apart;
transmit data indicative of the second potential difference (V₂);
measure a third potential difference (V₃) between a fifth electrode (124e) and a sixth electrode (124f) of the plurality of electrodes (124), the fifth electrode (124e) and the sixth electrode (124f) being spaced a third predetermined distance (d₃) apart; and
transmit data indicative of the third potential difference (V₃); and
wherein the storing comprises storing data indicative of the property of the electric field based at least in part on the data indicative of the first potential difference (V₁), the data indicative of the second potential difference (V₂), the data indicative of the third potential difference (V₃), the first predetermined distance (d₁), the second predetermined distance (d₂) and the third predetermined distance (d₃).

12. The system of claim 9, wherein the processor-executable code (188) of the computer system (172), when executed, is further configured to cause the processor (176) of the computer system (172) to:
responsive to receiving the data indicative of the orientation with the communication device (180) thereof, store the data indicative of the orientation.

13. The system of claim 9, wherein the processor-executable code (188) of the computer system (172), when executed, is further configured to cause the processor (176) of the computer system (172) to:
transmit, with the communication device (180) thereof, an instruction to
transition the probing device (116) into a measurement mode;
wherein the processor-executable code (144) of the probing device (116), when executed, is further configured to cause the processor (132) of the probing device (116) to:
responsive to receiving the instruction with the communication device (136) thereof, transition the probing device (116) into the measurement mode, wherein the measuring of the potential difference (Vₓ) and the transmitting of the data indicative of the potential difference (Vₓ) are performed when the probing device (116) is in the measurement mode; and
transition the probing device (116) into a standby mode.

14. The system of claim 13, wherein the processor-executable code (188) of the computer system (172), when executed, is further configured to cause the processor (176) of the computer system (172) to:
transmit, with the communication device (180) thereof, an instruction to transition the probing device (116) into the standby mode; and
wherein the processor-executable code (144) of the probing device (116), when executed, is further configured to cause the processor (132) of the probing device (116) to:
responsive to receiving the instruction to transition the probing device (116) into the standby mode, transition the probing device (116) into the standby mode.

## Patentansprüche

1. Verschluckbare oder implantierbare Sondierungsvorrichtung, die dazu ausgelegt ist, eine Eigenschaft von Tumortherapiefeldern, TTFields, zu bestimmen, die auf einen Patienten angewendet werden, umfassend:
ein Gehäuse (120), das eine biokompatible Außenfläche (129) aufweist;
einen oder mehrere Orientierungssensoren (168), die an einer bekannten Stelle innerhalb des Gehäuses (120) getragen werden;
eine Vielzahl von Elektroden (124), die von dem Gehäuse (120) getragen werden; und
eine innerhalb des Gehäuses (120) getragene Steuereinheit (128), wobei die Steuereinheit (128) einen Prozessor (132), eine Kommunikationsvorrichtung (136) und ein nichtflüchtiges computerlesbares Medium (140) umfasst, das vom Prozessor ausführbaren Code (144) speichert, der, wenn er ausgeführt wird, so konfiguriert ist, dass er den Prozessor (132) veranlasst zum:
Messen, mit mindestens einem des einen oder der mehreren Orientierungssensoren (168), einer Orientierung des mindestens einen des einen oder der mehreren Orientierungssensoren (168);
Messen einer Potentialdifferenz (Vₓ) zwischen einer ersten Elektrode (124a) und einer zweiten Elektrode (124b) der Vielzahl von Elektroden (124), wobei die erste Elektrode (124a) und die zweite Elektrode (124b) um einen vorbestimmten Abstand (dₓ) voneinander beabstandet sind; und
Übertragen, mit der Kommunikationsvorrichtung (136), von Daten, die die Potentialdifferenz (Vₓ) angeben, und Daten, die die Orientierung angeben.

2. Vorrichtung nach Anspruch 1, wobei der vom Prozessor ausführbare Code (144), wenn er ausgeführt wird, ferner so konfiguriert ist, dass er den Prozessor (132) veranlasst, mit der Kommunikationsvorrichtung (136) mindestens eine der Daten, die die Potentialdifferenz (Vₓ) angeben, und Daten, die eine Eigenschaft eines elektrischen Felds basierend mindestens teilweise auf den Daten, die die Potentialdifferenz (Vₓ), und dem vorbestimmten Abstand (dₓ) zu übertragen.

3. Vorrichtung nach Anspruch 2, wobei die erste Elektrode (124a) und die zweite Elektrode (124b) an antipodalen Punkten des Gehäuses (120) von der biokompatiblen Außenfläche (129) getragen werden und das Übertragen Übertragen mindestens einer der Daten, die die Potentialdifferenz (Vₓ) angeben, und Daten, die eine Stärke des elektrischen Felds basierend mindestens teilweise auf einem Quotienten angeben, der durch Dividieren der Daten, die die Potentialdifferenz (Vₓ) angeben, durch den vorbestimmten Abstand (dₓ) bestimmt wird, umfasst.

4. Vorrichtung nach Anspruch 1, wobei die Potentialdifferenz (Vₓ) eine erste Potentialdifferenz (V₁) ist, der vorbestimmte Abstand (dₓ) ein erster vorbestimmter Abstand (d₁) ist und der vom Prozessor ausführbare Code (144), wenn er ausgeführt wird, ferner so konfiguriert ist, dass er den Prozessor (132) veranlasst zum:
Messen einer zweiten Potentialdifferenz (V₂) zwischen einer dritten Elektrode (124c) und einer vierten Elektrode (124d) der Vielzahl von Elektroden (124), wobei die dritte Elektrode (124c) und die vierte Elektrode (124d) um einen zweiten vorbestimmten Abstand (d₂) voneinander beabstandet sind;
Messen einer dritten Potentialdifferenz (V₃) zwischen einer fünften Elektrode (124e) und einer sechsten Elektrode (124f) der Vielzahl von Elektroden (124), wobei die fünfte Elektrode (124e) und die sechste Elektrode (124f) um einen dritten vorbestimmten Abstand (d₃) voneinander beabstandet sind; und
Übertragen, mit der Kommunikationsvorrichtung (136), mindestens einer von Daten, die die zweite Potentialdifferenz (V₂) angeben, und Daten, die die dritte Potentialdifferenz (V₃) angeben.

5. Vorrichtung nach Anspruch 4, wobei der vom Prozessor ausführbare Code (144), wenn er ausgeführt wird, ferner so konfiguriert ist, dass er den Prozessor (132) veranlasst, mit der Kommunikationsvorrichtung (136) Daten zu übertragen, die eine Eigenschaft eines elektrischen Felds basierend mindestens teilweise auf den Daten, die die erste Potentialdifferenz (V₁) angeben, den Daten, die die zweite Potentialdifferenz (V₂) angeben, den Daten, die die dritte Potentialdifferenz (V₃) angeben, dem ersten vorbestimmten Abstand (d₁), dem zweiten vorbestimmten Abstand (d₂) und dem dritten vorbestimmten Abstand (d₃) angeben.

6. Vorrichtung nach Anspruch 4, wobei die erste Elektrode (124a) und die zweite Elektrode (124b) entlang einer ersten Achse (130a) des Gehäuses (120) von der biokompatiblen Außenfläche (129) getragen werden, die dritte Elektrode (124c) und die vierte Elektrode (124d) entlang einer zweiten Achse (130b) des Gehäuses (120) von der biokompatiblen Außenfläche (129) getragen werden, die fünfte Elektrode (124e) und die sechste Elektrode (124f) entlang einer dritten Achse (130c) des Gehäuses (120) von der biokompatiblen Außenfläche (129) getragen werden und die erste Achse (130a), die zweite Achse (130b) und die dritte Achse (130c) paarweise senkrecht zueinander stehen.

7. Vorrichtung nach Anspruch 1, wobei sie kugelförmig ist.

8. Vorrichtung nach Anspruch 1, die mindestens sechs Elektroden (124) umfasst, die optional paarweise gruppiert sind.

9. System, das umfasst:
die Sondierungsvorrichtung (116) nach einem der Ansprüche 1 bis 8; und
ein Computersystem (172), das einen Prozessor (176), eine Kommunikationsvorrichtung (180) und ein nichtflüchtiges computerlesbares Medium (184) umfasst, das vom Prozessor ausführbaren Code (188) speichert, der, wenn er ausgeführt wird, so konfiguriert ist, dass er den Prozessor (176) des Computersystems (172) veranlasst zum:
infolgedessen, dass die Daten, die die Potentialdifferenz (Vₓ) angeben, mit der Kommunikationsvorrichtung (180) davon empfangen werden, Speichern von Daten, die eine Eigenschaft eines elektrischen Felds basierend mindestens teilweise auf den Daten, die die Potentialdifferenz (Vₓ), und dem vorbestimmten Abstand (dₓ).

10. System nach Anspruch 9, wobei die erste Elektrode (124a) und die zweite Elektrode (124b) an antipodalen Punkten des Gehäuses (120) von der biokompatiblen Außenfläche (129) getragen werden und das Speichern Speichern von Daten umfasst, die eine Stärke des elektrischen Felds basierend mindestens teilweise auf einem Quotienten angeben, der durch Dividieren der Daten, die die Potentialdifferenz (Vₓ) angeben, durch den vorbestimmten Abstand (dₓ) bestimmt wird.

11. System nach Anspruch 9, wobei die Potentialdifferenz (Vₓ) eine erste Potentialdifferenz (V₁) ist, der vorbestimmte Abstand (dₓ) ein erster vorbestimmter Abstand (d₁) ist und der vom Prozessor ausführbare Code (144) der Sondierungsvorrichtung (116), wenn er ausgeführt wird, ferner so konfiguriert ist, dass er den Prozessor (132) der Sondierungsvorrichtung (116) veranlasst zum:
Messen einer zweiten Potentialdifferenz (V₂) zwischen einer dritten Elektrode (124c) und einer vierten Elektrode (124d) der Vielzahl von Elektroden (124), wobei die dritte Elektrode (124c) und die vierte Elektrode (124d) um einen zweiten vorbestimmten Abstand (d₂) voneinander beabstandet sind;
Übertragen von Daten, die die zweite Potentialdifferenz (V₂) angeben;
Messen einer dritten Potentialdifferenz (V₃) zwischen einer fünften Elektrode (124e) und einer sechsten Elektrode (124f) der Vielzahl von Elektroden (124), wobei die fünfte Elektrode (124e) und die sechste Elektrode (124f) um einen dritten vorbestimmten Abstand (d₃) voneinander beabstandet sind; und
Übertragen von Daten, die die dritte Potentialdifferenz (V₃) angeben; und
wobei das Speichern Speichern von Daten umfasst, die die Eigenschaft des elektrischen Felds basierend mindestens teilweise auf den Daten, die die erste Potentialdifferenz (V₁) angeben, den Daten, die die zweite Potentialdifferenz (V₂) angeben, den Daten, die die dritte Potentialdifferenz (V₃) angeben, dem ersten vorbestimmten Abstand (d₁), dem zweiten vorbestimmten Abstand (d₂) und dem dritten vorbestimmten Abstand (d₃) angeben.

12. System nach Anspruch 9, wobei der vom Prozessor ausführbare Code (188) des Computersystems (172), wenn er ausgeführt wird, ferner so konfiguriert ist, dass er den Prozessor (176) des Computersystems (172) veranlasst zum:
infolgedessen, dass die Daten, die die Orientierung angeben, mit der Kommunikationsvorrichtung (180) davon empfangen werden, Speichern der Daten, die die Orientierung angeben.

13. System nach Anspruch 9, wobei der vom Prozessor ausführbare Code (188) des Computersystems (172), wenn er ausgeführt wird, ferner so konfiguriert ist, dass er den Prozessor (176) des Computersystems (172) veranlasst zum:
Übertragen, mit der Kommunikationsvorrichtung (180) davon, einer Anweisung, die Sondierungsvorrichtung (116) in einen Messmodus zu versetzen;
wobei der vom Prozessor ausführbare Code (144) der Sondierungsvorrichtung (116), wenn er ausgeführt wird, ferner so konfiguriert ist, dass er den Prozessor (132) der Sondierungsvorrichtung (116) veranlasst zum:
infolgedessen, dass die Anweisung mit der Kommunikationsvorrichtung (136) davon empfangen wird, Versetzen der Sondierungsvorrichtung (116) in den Messmodus, wobei das Messen der Potenzialdifferenz (Vₓ) und das Übertragen der Daten, die die Potenzialdifferenz (Vₓ) angeben, durchgeführt werden, wenn sich die Sondierungsvorrichtung (116) im Messmodus befindet; und
Versetzen der Sondierungsvorrichtung (116) in einen Ruhemodus.

14. System nach Anspruch 13, wobei der vom Prozessor ausführbare Code (188) des Computersystems (172), wenn er ausgeführt wird, ferner so konfiguriert ist, dass er den Prozessor (176) des Computersystems (172) veranlasst zum:
Übertragen, mit der Kommunikationsvorrichtung (180) davon, einer Anweisung, die Sondierungsvorrichtung (116) in einen Ruhemodus zu versetzen; und
wobei der vom Prozessor ausführbare Code (144) der Sondierungsvorrichtung (116), wenn er ausgeführt wird, ferner so konfiguriert ist, dass er den Prozessor (132) der Sondierungsvorrichtung (116) veranlasst zum:
infolgedessen, dass die Anweisung, die Sondierungsvorrichtung (116) in den Ruhemodus zu versetzen, empfangen wird, Versetzen der Sondierungsvorrichtung (116) in den Ruhemodus.

## Revendications

1. Dispositif de sondage pouvant être ingéré ou implanté, conçu pour déterminer une propriété des champs de traitement tumoral, TTFields, appliqués à un patient, comprenant :
un boîtier (120) présentant une surface extérieure biocompatible (129) ;
un ou plusieurs capteurs d'orientation (168) supportés dans un emplacement connu à l'intérieur du boîtier (120) ;
une pluralité d'électrodes (124) supportées par le boîtier (120) ; et
un dispositif de commande (128) supporté à l'intérieur du boîtier (120), le dispositif de commande (128) comprenant un processeur (132), un dispositif de communication (136) et un support non transitoire lisible par ordinateur (140) stockant un code exécutable par le processeur (144) qui, lorsqu'il est exécuté, est configuré pour amener le processeur (132) à :
mesurer, avec au moins l'un des un ou plusieurs capteurs d'orientation (168), une orientation du au moins un des un ou plusieurs capteurs d'orientation (168) ;
mesurer une différence de potentiel (Vₓ) entre une première électrode (124a) et une deuxième électrode (124b) de la pluralité d'électrodes (124), la première électrode (124a) et la deuxième électrode (124b) étant espacées d'une distance prédéterminée (dₓ) ; et
transmettre, avec le dispositif de communication (136), des données indiquant la différence de potentiel (Vₓ) et des données indiquant l'orientation.

2. Dispositif selon la revendication 1, dans lequel le code exécutable par le processeur (144), lorsqu'il est exécuté, est configuré en outre pour amener le processeur (132) à transmettre, avec le dispositif de communication (136), au moins l'une des données indiquant la différence de potentiel (Vₓ) et des données indiquant une propriété d'un champ électrique sur la base au moins en partie des données indiquant la différence de potentiel (Vₓ) et la distance prédéterminée (dₓ).

3. Dispositif selon la revendication 2, dans lequel la première électrode (124a) et la deuxième électrode (124b) sont supportées par la surface extérieure biocompatible (129) aux points antipodaux du boîtier (120), et la transmission comprend la transmission d'au moins l'une des données indiquant la différence de potentiel (Vₓ) et des données indiquant une amplitude du champ électrique sur la base au moins en partie d'un quotient déterminé en divisant les données indiquant la différence de potentiel (Vₓ) par la distance prédéterminée (dₓ).

4. Dispositif selon la revendication 1, dans lequel la différence de potentiel (Vₓ) est une première différence de potentiel (V₁), la distance prédéterminée (dₓ) est une première distance prédéterminée (d₁), et le code exécutable par le processeur (144), lorsqu'il est exécuté, est configuré en outre pour amener le processeur (132) à :
mesurer une deuxième différence de potentiel (V₂) entre une troisième électrode (124c) et une quatrième électrode (124d) de la pluralité d'électrodes (124), la troisième électrode (124c) et la quatrième électrode (124d) étant espacées d'une deuxième distance prédéterminée (d₂) ;
mesurer une troisième différence de potentiel (V₃) entre une cinquième électrode (124e) et une sixième électrode (124f) de la pluralité d'électrodes (124), la cinquième électrode (124e) et la sixième électrode (124f) étant espacées d'une troisième distance prédéterminée (d₃) ; et
transmettre, avec le dispositif de communication (136), au moins l'une des données indiquant la deuxième différence de potentiel (V₂) et des données indiquant la troisième différence de potentiel (V₃).

5. Dispositif selon la revendication 4, dans lequel le code exécutable par le processeur (144), lorsqu'il est exécuté, est configuré en outre pour amener le processeur (132) à transmettre, avec le dispositif de communication (136), des données indiquant une propriété d'un champ électrique sur la base au moins en partie des données indiquant la première différence de potentiel (V₁), des données indiquant la deuxième différence de potentiel (V₂), des données indiquant la troisième différence de potentiel (V₃), de la première distance prédéterminée (d₁), de la deuxième distance prédéterminée (d₂) et de la troisième distance prédéterminée (d₃).

6. Dispositif selon la revendication 4, dans lequel la première électrode (124a) et la deuxième électrode (124b) sont supportées par la surface extérieure biocompatible (129) le long d'un premier axe (130a) du boîtier (120), la troisième électrode (124c) et la quatrième électrode (124d) sont supportées par la surface extérieure biocompatible (129) le long d'un deuxième axe (130b) du boîtier (120), la cinquième électrode (124e) et la sixième électrode (124f) sont supportées par la surface extérieure biocompatible (129) le long d'un troisième axe (130c) du boîtier (120), et le premier axe (130a), le deuxième axe (130b) et le troisième axe (130c) sont perpendiculaires deux à deux.

7. Dispositif selon la revendication 1, de forme sphérique.

8. Dispositif selon la revendication 1, comprenant au moins six électrodes (124), facultativement regroupées par paires.

9. Système comprenant :
le dispositif de sondage (116) selon l'une quelconque des revendications 1 à 8 ; et
un système informatique (172) comprenant un processeur (176), un dispositif de communication (180) et un support non transitoire lisible par ordinateur (184) stockant un code exécutable par le processeur (188) qui, lorsqu'il est exécuté, est configuré pour amener le processeur (176) du système informatique (172) à :
en réponse à la réception des données indiquant la différence de potentiel (Vₓ) avec le dispositif de communication (180) de celui-ci, stocker des données indiquant une propriété d'un champ électrique sur la base au moins en partie des données indiquant la différence de potentiel (Vₓ) et la distance prédéterminée (dₓ).

10. Système selon la revendication 9, dans lequel la première électrode (124a) et la deuxième électrode (124b) sont supportées par la surface extérieure biocompatible (129) aux points antipodaux du boîtier (120), et le stockage comprend le stockage de données indiquant une amplitude du champ électrique sur la base au moins en partie d'un quotient déterminé en divisant la différence de potentiel (Vₓ) par la distance prédéterminée (dₓ).

11. Système selon la revendication 9, dans lequel la différence de potentiel (Vₓ) est une première différence de potentiel (V₁), la distance prédéterminée (dₓ) est une première distance prédéterminée (d₁), et le code exécutable par le processeur (144) du dispositif de sondage (116), lorsqu'il est exécuté, est configuré en outre pour amener le processeur (132) du dispositif de sondage (116) à :
mesurer une deuxième différence de potentiel (V₂) entre une troisième électrode (124c) et une quatrième électrode (124d) de la pluralité d'électrodes (124), la troisième électrode (124c) et la quatrième électrode (124d) étant espacées d'une deuxième distance prédéterminée (d₂) ;
transmettre des données indiquant la deuxième différence de potentiel (V₂) ;
mesurer une troisième différence de potentiel (V₃) entre une cinquième électrode (124e) et une sixième électrode (124f) de la pluralité d'électrodes (124), la cinquième électrode (124e) et la sixième électrode (124f) étant espacées d'une troisième distance prédéterminée (d₃) ; et
transmettre des données indiquant la troisième différence de potentiel (V₃) ; et
dans lequel le stockage comprend le stockage de données indiquant la propriété du champ électrique sur la base au moins en partie des données indiquant la première différence de potentiel (V₁), des données indiquant la deuxième différence de potentiel (V₂), des données indiquant la troisième différence de potentiel (V₃), de la première distance prédéterminée (d₁), de la deuxième distance prédéterminée (d₂) et de la troisième distance prédéterminée (d₃).

12. Système selon la revendication 9, dans lequel le code exécutable par le processeur (188) du système informatique (172), lorsqu'il est exécuté, est configuré en outre pour amener le processeur (176) du système informatique (172) à :
en réponse à la réception des données indiquant l'orientation avec le dispositif de communication (180) de celui-ci, stocker les données indiquant l'orientation.

13. Système selon la revendication 9, dans lequel le code exécutable par le processeur (188) du système informatique (172), lorsqu'il est exécuté, est configuré en outre pour amener le processeur (176) du système informatique (172) à :
transmettre, avec le dispositif de communication (180) de celui-ci, une instruction pour faire passer le dispositif de sondage (116) en mode de mesure ;
dans lequel le code exécutable par le processeur (144) du dispositif de sondage (116), lorsqu'il est exécuté, est configuré en outre pour amener le processeur (132) du dispositif de sondage (116) à :
en réponse à la réception de l'instruction avec le dispositif de communication (136) de celui-ci, faire passer le dispositif de sondage (116) en mode de mesure, dans lequel la mesure de la différence de potentiel (Vₓ) et la transmission des données indiquant la différence de potentiel (Vₓ) sont réalisées lorsque le dispositif de sondage (116) est en mode de mesure ; et
faire passer le dispositif de sondage (116) en mode veille.

14. Système selon la revendication 13, dans lequel le code exécutable par le processeur (188) du système informatique (172), lorsqu'il est exécuté, est configuré en outre pour amener le processeur (176) du système informatique (172) à :
transmettre, avec le dispositif de communication (180) de celui-ci, une instruction pour faire passer le dispositif de sondage (116) en mode veille ; et
dans lequel le code exécutable par le processeur (144) du dispositif de sondage (116), lorsqu'il est exécuté, est configuré en outre pour amener le processeur (132) du dispositif de sondage (116) à :
en réponse à la réception de l'instruction de faire passer le dispositif de sondage (116) en mode veille, faire passer le dispositif de sondage (116) en mode veille.
